# EUROPEAN PATENT APPLICATION

(11) **EP 4 024 258 A1**
(43) Date of publication of application: **06.07.2022**
(21) Application number: 21215124.5
(22) Date of filing: 16.12.2021
(51) Int. Cl.: G06F 30/13, G06F 30/25, G06F 30/28, G06F 111/10

(54) **CONTAMINANT SPREAD ANALYSIS**

(30) Priority: 31.12.2020 US 202017139238
(71) Applicant: Trane International Inc., Davidson, NC 28036 (US)
(72) Inventor: LIU, Yi, Concord, North Carolina, 28027 (US); WANG, Gang, Holmen, Wisconsin, 54636 (US); LIEBEL, Stephanie Veronica, Nashville, Tennessee, 37206 (US); POLYZOIS, Christos Alkiviadis, Bloomington, Minnesota, 55438 (US); PETERS, Michael, Mooresville, North Carolina, 28115 (US); YODER, Katheryn, La Crosse, Wisconsin, 54601 (US)
(74) Representative: Haseltine Lake Kempner LLP

(57) **Abstract**

A contaminant-risk analysis device generates a CAD model of a defined space based on received parameters, simulates a contaminant spread model relative to the CAD model, generates a contaminant risk assessment for the defined space based on the contaminant spread model being applied to the CAD model, and generates a revised CAD model of the defined space based on the generated contaminant risk assessment.

## Description

### TECHNICAL FIELD

The embodiments described herein pertain generally to assessing the potential spread of a contaminant within a defined space.

### BACKGROUND

Lessons learned from the pandemic of 2019 - 20, caused by the virus known to some as "Covid-19," include a heightened awareness of the quality and/or flow of air within a given space, although proof of the air quality within the given space is typically unsubstantiated.

### SUMMARY

In one example embodiment, a computer-readable medium stores executable components that, when executed, cause one or more processing devices to perform a contaminant-risk analysis. The components include an interface to receive parameters of a defined space from a user device, a computer-aided design (CAD) generator to generate a CAD model of the defined space based on the received parameters, and a simulator. The simulator is to generate a visual simulation of airflow within the CAD model of the defined space, a visual simulation of a flow of contaminants within the CAD model of the defined space, and a visual simulation of a flow of disinfectants within the CAD model of the defined space. Variations in velocity of the airflow as well as velocity and/or density of contaminants and disinfectants are depicted in accordance with corresponding color-coding scales. The components also include a risk assessment generator to simultaneously project the visual simulation of the airflow, the flow of contaminants, and the flow disinfectants onto the CAD model of the defined space.

In another example embodiment, a risk-assessment system comprises a user device on which a version of an application is executing, and a service provider that has at least one processor communicatively connected to the application executing on the user device. The processor generates a CAD model of the defined space using the received parameters, generates a visual simulation of airflow within the CAD model of the defined space, generates a visual simulation of a flow of contaminants within the CAD model of the defined space, generates a visual simulation of a flow of disinfectants within the CAD model of the defined space, and projects the visual simulation of the airflow, the visual simulation of the flow of contaminants, and the visual simulation of the flow of disinfectants onto the CAD model of the defined space.

In yet another example embodiment, a computer-readable medium stores executable instructions that, when executed, cause one or more processing devices to perform a contaminant-risk analysis. The instructions include receiving parameters of a defined space input by a user device, with the parameters including dimensions of the defined space, placement of at least one of an air inlet or an air outlet in the defined space, and placement of at least one contaminant mitigation device in the defined space. The instructions also include generating a CAD model of the defined space based on the parameters, generating a visual simulation of airflow within the CAD model of the defined space, generating a visual simulation of a flow of contaminants within the CAD model of the defined space, generating a visual simulation of a flow of disinfectants within the CAD model of the defined space, and producing a graphical risk assessment of the defined space by simultaneously projecting the visual simulations of the airflow, the flow of contaminants, and the flow of disinfectants onto the CAD model of the defined space.

### BRIEF DESCRIPTION OF THE DRAWINGS

In the detailed description that follows, embodiments are described as illustrations only since various changes and modifications will become apparent to those skilled in the art from the following detailed description. The use of the same reference numbers in different figures indicates similar or identical items.
**FIG.** 1 shows a block diagram representing an example contaminant risk assessment system, arranged in accordance with at least some embodiments described and recited herein;
**FIGS. 2A** - **2C** show example representations of defined spaces for which a contaminant risk assessment is provided, in accordance with at least some embodiments described and recited herein;
**FIG. 3** shows an example processing flow for implementing a contaminant risk assessment, in accordance with at least some embodiments described and recited herein;
**FIG. 4** shows an illustrative computing embodiment, in which aspects of the contaminant risk assessment may be implemented as executable instructions stored on a computer-readable medium, in accordance with at least some embodiments described and recited herein.

### DETAILED DESCRIPTION

In the following detailed description, reference is made to the accompanying drawings, which form a part of the description. In the drawings, similar symbols typically identify similar components, unless context dictates otherwise. Furthermore, unless otherwise noted, the description of each successive drawing may reference features from one or more of the previous drawings to provide clearer context and a more substantive explanation of the current example embodiment. Still, the example embodiments described in the detailed description, drawings, and claims are not intended to be limiting. Other embodiments may be utilized, and other changes may be made, without departing from the spirit or scope of the subject matter presented herein. It will be readily understood that the aspects of the present disclosure, as generally described herein and illustrated in the drawings, may be arranged, substituted, combined, separated, and designed in a wide variety of different configurations, all of which are explicitly contemplated herein.

In the present description and recitation, the following terms may be used, in addition to their accepted meaning, as follows:

Contaminant may refer to one or more infectious agents that are capable of growth and multiplication in living cells, capable of causing diseases in humans, animals, or plants. As referenced herein, a contaminant may include, but not be limited to one or more of a virus, microbiological, pathogen, bacteria, fungus, and may be referenced interchangeably.

Disinfectant may refer to a chemical agent that may be used to destroy, deactivate, or significantly reduce the concentration of contaminants. As referenced herein, a disinfectant may include, but not be limited to, one or more chemical agents, ultraviolet (UV) light or UV radiation.

CAD geometry may refer to computer-aided design geometry, which is but a representative modeling technique utilized by the example embodiments described and recited herein.

CFD may refer to computational fluid dynamics, by which airflow pattern and contaminant spread inside a defined space may be simulated.

DHP may refer to dry hydrogen peroxide, generators of which may produce air having dilute, dry, non-aqueous hydrogen peroxide from ambient air.

PCO may refer to photocatalytic oxidation, by which free radicals are generated by exposing a catalyst to an energy source to destroy airborne contaminants. In accordance with the embodiments described and recited herein, PCO may be implemented in a suitable apparatus, device, and the like, including, but not limited to, an air purifier, ultraviolet light source, and the like.

As set forth above, described and recited herein are methods, programs, systems, apparatuses, and components pertaining to contaminant spread analysis and corresponding risk analysis for a defined space.

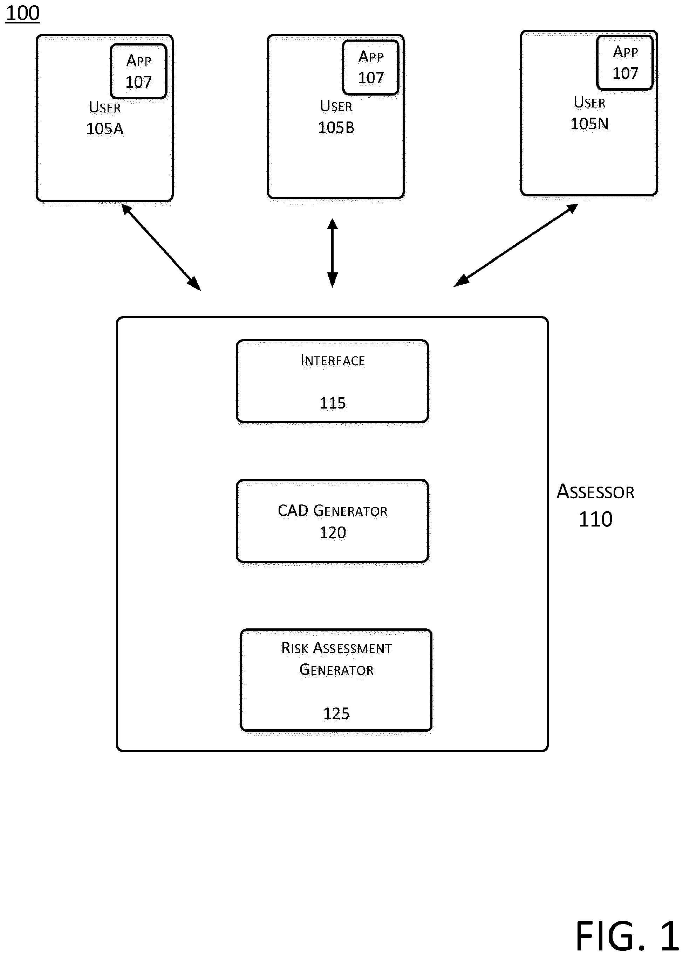

**FIG. 1** shows a block diagram representing an example contaminant risk assessment system, arranged in accordance with at least some embodiments described and recited herein. As depicted, system 100 includes, at least, plural users 105A - 105N and assessor 110. Assessor 110 may include, at least, interface 115, CAD generator 120, and risk assessment generator 125.

User devices 105A - 105N may refer to processing devices in the form of any one of a smart phone, tablet computer, notebook computer, personal computer, and/or personal communication terminal, configured to be communicatively coupled to at least assessor 110 over at least one of a wired or wireless link or a LAN or WAN connection, serially or in parallel.

Application 107 may refer to an instance, or version, of an application that may be programmed, designed, and/or configured to be installed and executed upon user devices 105 to facilitate interactive communication with assessor 110. Additionally, or alternatively, application 107 may be implemented as a web browser that facilitates interactive communication between the respective user device 105 and assessor 110.

Application 107 may refer to an instance, or version, of a measurement application that may be programmed, designed, and/or configured to work in conjunction with a camera installed on or connected to user device 105 to capture the parameters, e.g., dimensions, of a defined space. The parameters may include, but not be limited to, the height and/or length of any portion of the defined space. Further, in cooperation with the camera, the application may capture dimensions of portions of the defined space relative to objects therein. As a non-limiting example, having captured a picture of a portion of the defined space, the measurement application 107 may measure dimensions of an object within the room, as well as measure dimensions relative to the object. Further to the example, the measurement application 107 may measure the height and length of a piece of furniture or even a person within a room, as well as measure the distance from the piece of furniture or person to one or more walls within the room, the distance from the piece of furniture or person to the ceiling of the room, the distance from the piece of furniture or person to an air inlet and/or air outlet, and the distance from the piece of furniture or person to a contaminant mitigation device.

Further, in accordance with at least one example embodiment described and/or recited herein, the parameters of a defined space may include dimensions of the defined space and/or placement of air inlets and/or air outlets within the defined space. Thus, for the embodiments described and recited herein, doors, windows, ceiling fans, box fans, stand-alone fans, *etc.,* are to be considered as either or both an air inlet or an air outlet.

Additionally, or alternatively, the placement of the air inlets or outlets may be received from a graphic user interface hosted by application 107, with the air inlets and air outlets being graphically depicted within a graphic model of the defined space. Further still, the parameters may further include the velocity, *i.e.,* rate of air into the defined space and the rate of air expelled from the defined space, both measured in, e.g.**,** CFM (cubic-feet-per-minute), which also may be determined by components of application 107 or, alternatively, may be otherwise determined or procured separate from application 107.

In accordance with at least one other example embodiment described and/or recited herein, application 107, in cooperation with the camera installed on or connected to user device 105, may produce a scanned 3-D image of the defined space.

The parameters of a defined space may further include preferred, probable, or likely quantity and/or placement of objects including, but not limited to, furniture and/or people within the defined space. The placement of the objects within the defined space may also be graphically depicted within a graphic model of the defined space.

Application 107 may be programmed, designed, and/or configured to transmit or otherwise facilitate the input of the measured or otherwise captured parameters of a defined space to assessor 110.

Although FIG. 1 shows only three representations of user devices 105, the quantity of such devices that may be utilized and/or implemented in the contaminant spread analysis and corresponding risk analysis, as described and recited herein, is in no way limited thereto. That is, as at least one embodiment of assessor 110 may include or utilize a quantum processor or super-computing cluster, the value "N" corresponding to user device 105N may be in the order of tens, hundreds, or even thousands in accordance with various embodiments described and recited herein. Yet, unless context otherwise requires, reference may be made herein to plural user devices 105 or to a singular user device 105, to the extent that each of user devices 105A - 105N have similar functions and/or operations.

Assessor 110 may refer to a service provider for providing, at least, a graphical contaminant spread and mitigation analysis. Assessor 110 may include a server or processing device that provides any of a variety of data and/or functionality to user devices 105, being communicatively connected to user devices 105 by way of a wired or wireless connection via a network (not shown).

Assessor 110 may be programmed, designed, and/or configured to provide contaminant risk assessment services to one or more of user devices 105, and access to assessor 110, for any one of user devices 105 may require a secured log-in.

As depicted, assessor 110 may include, at least, interface 115, CAD generator 120, and risk assessment generator 125.

Interface 115 may be programmed, designed, and/or configured to interactively communicate with the instance of application 107 that is executing on user device 105. Accordingly, interface may receive, from respective versions of application 107 running on one or more of user devices 105 either serially or in parallel, the parameters of a defined space input by respective user device 105, as described above, via application 107. In accordance with the above description, the parameters of the defined space may include a listing of dimensions corresponding to portions of the defined space as well as pictures of portions of the defined space. Even further, the pictures of portions of the defined space may include pictures captured by a camera installed on or connected to user device 105, blueprints or a floorplan corresponding to a defined space that has been uploaded or otherwise accessed by user device 105, *etc.*

In accordance with at least some example embodiments described and recited herein, a defined space may pertain to one or more rooms within a building, a warehouse, an automobile, a bus, and the like, so long as measurements may be articulated or a configuration of the space may be graphically depicted.

Further, interface 115 may be programmed, designed, and/or configured to interactively communicate with multiple instances of application 107 that are executing on respective user devices 105A in serial or in parallel. Thus, in accordance with other example embodiments described and recited herein, interface 115 may receive the parameters of multiple defined spaces, e.g., rooms, within a single building or structure.

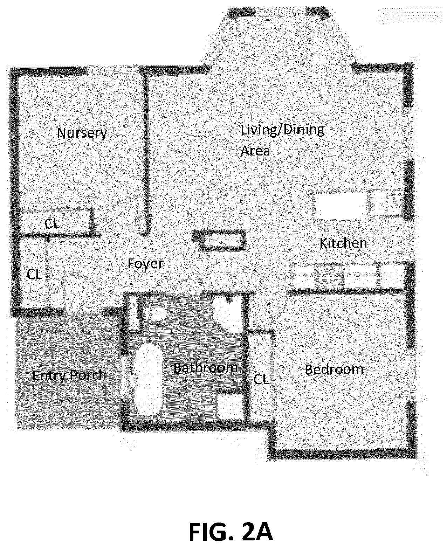

CAD generator 120 may be programmed, designed, and/or configured to receive or generate a CAD model of a defined space based on the parameters received by interface 115. Non-limiting examples of such a generated CAD model are shown in FIGS. 2A - 2C.

CAD generator 120 may be programmed, designed, and/or configured to generate a CAD model of a defined space based on the received parameters or as part of an interactive process by which user input, via application 107 running on user device 105, may be provided graphically. Graphic input may include the interactive placement of the parameters and/or objects relative to a graphical user interface having a graphic representation, e.g., CAD model, of the defined space. Further, graphic input may be provided on a continual basis as parameters change for a particular defined space, for example, if furniture is moved and/or when a number of people within the defined space changes.

CAD generator 120 may generate CAD models by receiving an uploaded 3-D scan of the defined space with listed parameters which may be converted into a 3-D CAD model. In accordance with at least one alternative embodiment, the parameters of the defined space may be input to CAD generator 120 to automatically create the model using the provided parameters.

The input parameters pertain to measurements for walls, floors, and ceiling of the defined space, as well as to the location of vents and one or more of furniture, one or more persons, and/or any measurable object in the defined space. CAD generator 120 may include or have access to a library of models for furniture and people that the user may interactively select, via a user interface, to represent a current or even likely configuration of the defined space. Thus, models or graphic representations of furniture and people may be interactively placed within a graphic representation of the defined space. Models or graphic representations of contaminant mitigation devices may also be interactively placed within the defined space. In accordance with at least some example embodiments described and/or recited herein, the aforementioned library may also store models or graphic representations of various vent geometries for inlet and outlet of an HVAC system for interactive placement in the defined space.

Risk assessment generator 125 may be programmed, designed, and/or configured to simulate airflow across the generated CAD model corresponding to the defined space. The simulation includes a graphical representation of air from the one or more air inlets to the defined space, a graphical representation of air to the one or more air outlets from the defined space, and the flow of air as it circulates within the defined space, including the flow of air around objects, in and out of doors and/or windows, off the floor, ceiling, and walls. The simulation may be provided to requesting user 105, via interface 115 and/or app 107.

Representations of velocity of the airflow relative to the generated CAD model may be made in accordance with a color-coding scale designated therefor. The airflow throughout the defined space may be represented by arrows, which may or may not be color-coded. The velocity of airflow may be represented by variations in the color-coding, following the arrows depicting the airflow. A scale or key that corresponds the variations in the color-coding to variations in the velocity of the airflow within the defined space may be displayed on the user device, via application 107.

Risk assessment generator 125 may further be programmed, designed, and/or configured to simulate a contaminant spread model across the generated CAD model corresponding to the defined space. The simulation, which may include a CFD (computational fluid dynamics) simulation, includes a designation of a viral or other contaminant source, e.g., person, who is graphically represented in the graphic representation of the defined space. That is, risk assessment generator 125 may simulate the circulation of the virus or other contaminants through the CAD model of the defined space, including the flow thereof off and around objects, through one or more doors and/or windows, off the floor, ceiling, and walls. The simulation may be provided to requesting user device 105, via interface 115 and/or app 107.

Representations of velocity and density of the contaminant relative to the generated CAD model may be made in accordance with a color-coding scale designated for, at least, the virus(es) and/or other contaminant(s). The flow thereof throughout the defined space may be represented by arrows, which may or may not be color-coded. The velocity of the flow of the virus or other contaminant may be represented by variations in the color-coding, following the arrows depicting the flow thereof. The density of the virus or other contaminant in portions of the defined space may also be represented by variations in the color-coding. A scale or key that corresponds to the variations in the color-coding to variations in the velocity and density of the virus or other contaminant within the defined space may be displayed on the user device, via application 107.

Risk assessment generator 125 may further be programmed, designed, and/or configured to simulate a flow of disinfectant or other contaminant-mitigant across the generated CAD model corresponding to the defined space. The simulation includes a graphical representation of disinfectant or mitigant from one or more contaminant-mitigating devices located within the defined space as the disinfectant or mitigant circulates within the defined space, including the flow thereof around objects, off the floor, ceiling, and walls. The simulation may be provided to requesting user 105, via interface 115 and/or app 107.

Representations of velocity and density of the disinfectant or mitigant relative to the generated CAD model may be made in accordance with a color-coding scale designated for, at least, the disinfectant or mitigant. The flow thereof throughout the defined space may be represented by arrows, which may or may not be color-coded. The velocity of the flow of the disinfectant or mitigant may be represented by variations in the color-coding, following the arrows depicting the flow thereof. The density of the disinfectant or mitigant in portions of the defined space may also be represented by variations in the color-coding. A scale or key that corresponds the variations in the color-coding to variations in the velocity and density of the disinfectant or mitigant within the defined space may be displayed on the user device, via application 107.

In accordance with at least one or more example embodiments described and recited herein, an ultra-violet (UV) light source may be included as a non-limiting example of a contaminant-mitigating device. UV light has contaminant-killing properties and, therefore, may be utilized as a disinfectant in accordance with the embodiments described and recited herein. Further, the propagation of UV light may be simulated by risk assessment generator 125, with the intensity thereof being represented by variations in the corresponding color-coding scale.

Risk assessment generator 125 may further be programmed, designed, and/or configured to project the simulated contaminant spread model, the simulated airflow, and the simulated flow of the disinfectant or mitigant, simultaneously, across the CAD model corresponding to the defined space. Accordingly, risk assessment generator 125 may produce a simulation, relative to the generated CAD model, that shows an airflow, velocity and density of a virus and/or contaminant, and velocity and density of a disinfectant, interacting relatively within a common CAD model to provide a color-coded assessment of contaminant risks as well as decontaminating needs and opportunities relative to the defined space.

Taking into consideration the input of air via the air inlets, the output of air via the air outlets, and the resulting flow of air circumventing the objects throughout the defined space, as well as the flow of a contaminant and the flow of a disinfectant or mitigant, risk assessment generator 125 may further generate a risk assessment for the defined space, starting with the contaminant source. Further still, if a score or other quantification of the generated risk assessment meets or exceeds a predetermined threshold, risk assessment generator 125 may generate a revision to the CAD model in which the contaminant spread model results in a risk assessment that is below the predetermined threshold.

Risk assessment generator 125 may be further programmed, designed, and/or configured to generate a revised CAD model for multiple defined spaces within a particular building or otherwise within close proximity, simultaneously, taking into account an effect of the airflow of one defined space on the airflow of one or more proximate defined spaces. The revised CAD model may also show a simulation, relative to the generated CAD model, that shows an improved airflow, decreased velocity and/or density of a contaminant, and increased velocity and/or density of a disinfectant, interacting relatively within the common CAD model to provide a color-coded recommendation to mitigate contaminant risks relative to the defined space.

The generated risk assessment may include, *e.g.,* a graphic and/or written description of the contaminant flow throughout the defined space including, but not limited to, a pathway of the contaminant flow and a rate of the contaminant flow.

The revised CAD model may, in accordance with the generated risk assessment, include revised placements of one or more objects within the defined space, recommendations for changes to the rate of the input of air via one or more air inlets and/or the rate of air output via one or more air outlets, quantity and/or placement of one or more mitigation devices within the defined space. Non-limiting examples of such mitigation devices may include DHP (dry hydrogen peroxide) generators and/or photocatalytic oxidation (PCO) air purifiers and the like.

Further, the generated CAD model may or may not include mitigation devices. Thus, if the generated CAD model does include one or more mitigation devices, the revised CAD model may include revised placement thereof; or the revised CAD model may maintain the original placement thereof. However, if the generated CAD model does not include a mitigation device, the revised CAD model may include placement of one or more mitigation devices.

Interface 115 may be further programmed, designed, and/or configured to transmit either or both of the generated risk assessment for a defined space and at least one revised CAD model to user device 105.

**FIGS. 2A** - **2C** show example representations of defined spaces for which a contaminant risk assessment is provided, in accordance with at least some embodiments described and recited herein. As depicted,

**FIG. 2A** shows a non-limiting example layout of a defined space that is generated in accordance with the embodiments described and recited herein. As a non-limiting example of the embodiments described and recited herein, FIG. 2A presents a listing of dimensions corresponding to portions of the defined space in the form of a floorplan. Other non-limiting examples may include pictures of portions of the defined space captured by a camera installed on or connected to user device 105 or blueprints that have been uploaded or otherwise accessed by user device 105, *etc.*

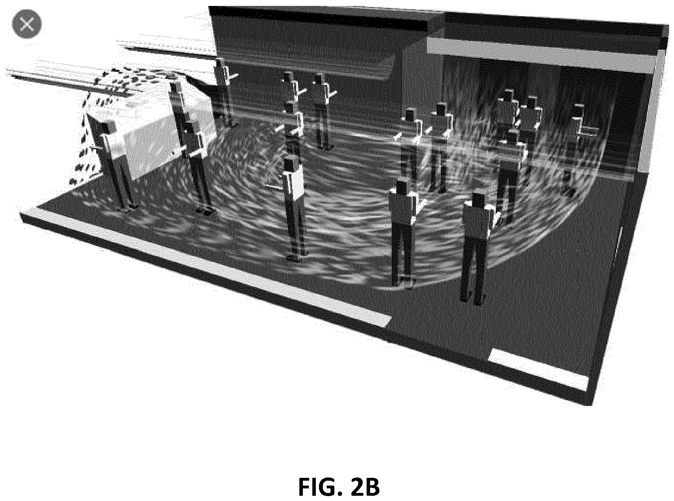

**FIG. 2B** shows a non-limiting example of an air velocity pattern applied to a CAD model of a defined space that is simulated in accordance with the embodiments described and recited herein. In at least one example embodiment, a high velocity area in which a contaminant transfer risk exceeds a predetermined level may be shown in, e.g., red; whereas a low velocity area in which the contaminant transfer risk is below the predetermined level may be shown in, e.g., blue.

As described and recited herein, risk assessment generator 125 may simulate a contaminant spread model across the generated CAD model corresponding to the defined space, simulate airflow across the generated CAD model corresponding to the defined space, and simulate a flow of disinfectant or other contaminant-mitigant across the generated CAD model corresponding to the defined space. Whereas all three simulations may be superimposed on top of each other, the simulations may also be produced individually, as presented in the non-limiting example of FIG. 2B.

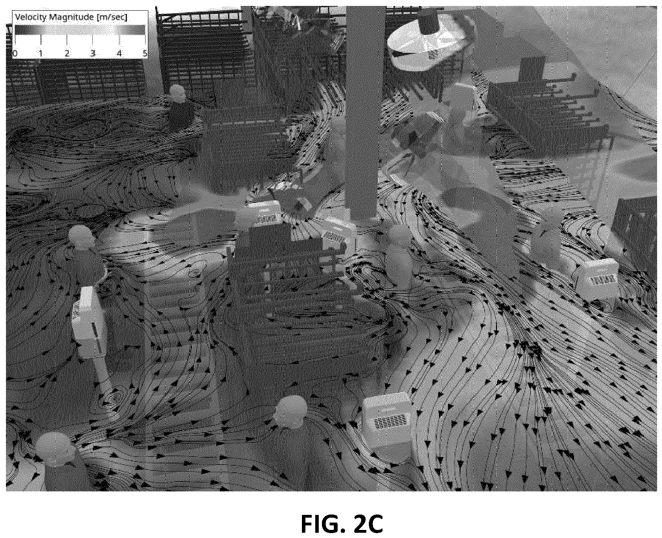

**FIG. 2C** shows a non-limiting example of a CAD model of a defined space in accordance with the embodiments described and recited herein after a risk analysis and/or evaluation has been generated. Based on a recommendation for altering one or more parameters for the defined space, the model may show a contaminant filtered with a percentage reduction per pass, thus the overall contaminant treatment of the room may be calculated.

As described and recited herein, risk assessment generator 125 may simulate the density of one or more contaminants through portions of the CAD model of the defined space as well as the density of the disinfectant or contaminant-mitigant through portions of the CAD model of the defined space. Whereas both simulations may be superimposed on top of each other as well as on top of the depictions of velocity as in FIG. 2B, the simulations may also be produced individually, as presented in the non-limiting example of FIG. 2C. Further, the representations of density may be presented in accordance with a designated color-coding scale, as in FIG. 2C, for the contaminant and/or the disinfectant.

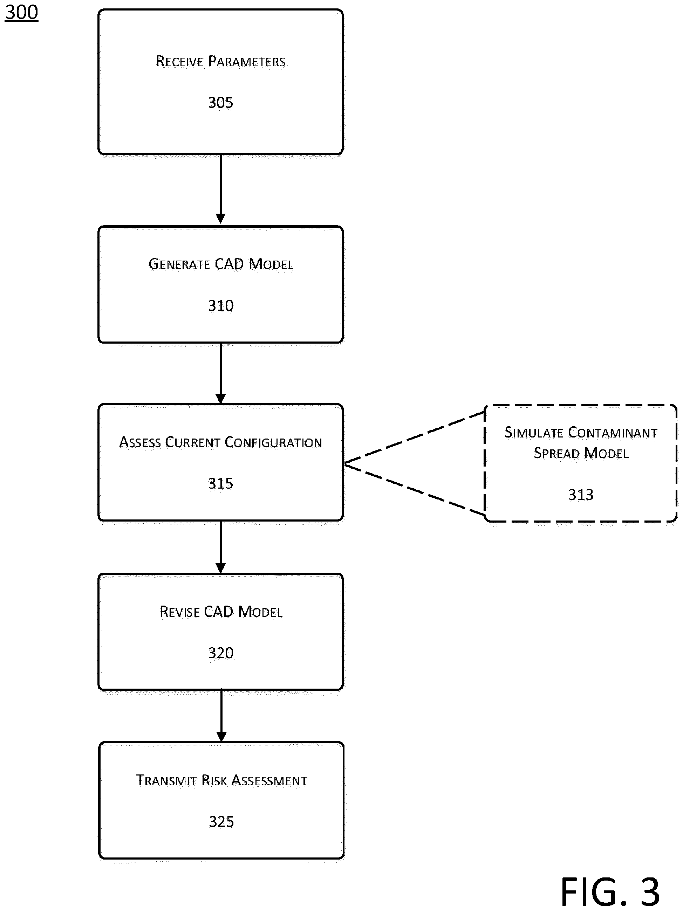

**FIG. 3** shows an example processing flow for implementing a contaminant risk assessment, in accordance with at least some embodiments described and recited herein. As depicted, processing flow 300 includes blocks 305, 310, 315, 320, and 325. Processing may begin at block 305.

At block 305 (Receive Parameters), interface 115 may interactively communicate with the instance of application 107 that is executing on user device 105 to receive, from one or more of user devices 105 either serially or in parallel, parameters of a defined space input by respective user device 105, via application 107. Accordingly, interface 115 may receive the parameters of multiple defined spaces, e.g., rooms, within a single building or structure. Processing may proceed from block 305 to block 310.

At block 310 (Generate CAD Model) CAD generator 120 may generate a CAD model for a defined space based on parameters received by interface 115. In accordance with other non-limiting example embodiments, the CAD model for the defined space may be generated based on interactive user input via application 107 executing on user device 105. Processing may proceed from block 310 to block 315.

At block 315 (Assess Current Configuration) risk assessment generator 125 may assess a contaminant spread risk for persons within or proximate to a defined space by simulating 313 a contaminant spread model relative to the generated CAD model corresponding to the defined space, starting with a contaminant source, e.g., person, that may be included as one of the input parameters, thereby generating a risk assessment for the defined space. Processing may proceed from block 315 to block 320.

At block 320 (Revise CAD Model), if a score or other quantification of the generated risk assessment meets or exceeds a predetermined threshold, risk assessment generator 125 may create a revised CAD model for which the contaminant spread model results in a risk assessment that is below the predetermined threshold. Similarly, risk assessment generator 125 may generate a revised CAD model for multiple defined spaces within a particular building or otherwise within close proximity, simultaneously, taking into account an effect of the airflow of one defined space on the airflow of a proximate defined space. Processing may proceed from block 320 to block 325.

The generated risk assessment may include, e.g., a graphic and/or written description of the contaminant flow throughout the defined space including, but not limited to, a pathway of the contaminant flow and a rate of the contaminant flow. Further, the analysis may include a recommendation of placement of one or more contaminant mitigation devices, non-limiting examples of which devices may include DHP (dry hydrogen peroxide) generators, photocatalytic oxidation (PCO) air purifiers, and the like, within the defined space, to reduce or maintain the contaminant spread risk below the predetermined level.

The revised CAD model may, in accordance with the generated risk assessment, include revised placements of one or more objects within the defined space, recommendations for changes to the rate of air input via one or more air inlets and/or the rate of air output via one or more air outlets, repositioning of one or more air inlets and/or outlets, placement of one or more of the aforementioned contaminant mitigation devices, non-limiting examples of which devices may include DHP (dry hydrogen peroxide) generators, photocatalytic oxidation (PCO) air purifiers, bipolar ionization (BPI) devices, ultraviolet (UV) lights, and the like, within the defined space.

At block 325 (Transmit Risk Assessment), interface 115 may transmit either or both of the generated risk assessment for a defined space and the revised CAD model to user device 105.

Accordingly, based on the description above, users may receive instant recommendations for reconfiguration of a defined space, *e.g.,* a room, multiple rooms, vehicle, *etc.,* based on user input, to reduce or even avoid the spread of a contaminant within the defined space.

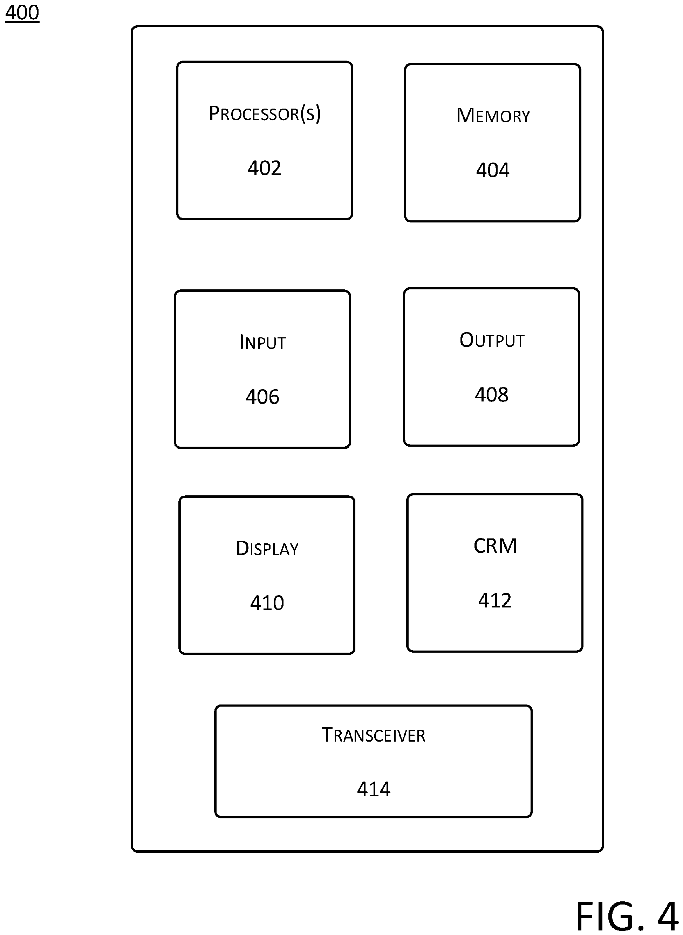

**FIG. 4** shows an illustrative computing embodiment, in which aspects of the contaminant risk assessment may be implemented as executable instructions stored on a computer-readable medium, in accordance with at least some embodiments described and recited herein. The computer-readable instructions may, for example, be executed by a processor of a device, as referenced herein, having a network element and/or any other device corresponds to thereto, particularly as applicable to the applications and/or programs described above corresponding to system 100 for contaminant risk assessment.

In a very basic configuration, a computing device 400 may typically include, at least, one or more processors 402, a system memory 404, one or more input components 406, one or more output components 408, a display component 410, a computer-readable medium 412, and a transceiver 414.

Processor 402 may refer to, *e.g.,* a microprocessor, a microcontroller, a digital signal processor, or any combination thereof, akin to assessor 110 described in accordance with FIG. 1.

Memory 404 may refer to, *e.g.,* a volatile memory, non-volatile memory, or any combination thereof. Memory 404 may store, therein, an operating system, an application, and/or program data. That is, memory 404 may store executable instructions to implement any of the functions or operations described above and, therefore, memory 404 may be regarded as a computer-readable medium.

Input component 406 may refer to a built-in or communicatively coupled keyboard, touch screen, or telecommunication device. Alternatively, input component 406 may include a microphone that is configured, in cooperation with a voice-recognition program that may be stored in memory 404, to receive voice commands from a user of computing device 400. Further, input component 406, if not built-in to computing device 400, may be communicatively coupled thereto via short-range communication protocols including, but not limited to, radio frequency or Bluetooth.

Output component 408 may refer to a component or module, built-in or removable from computing device 400, which is configured to output commands and data to an external device.

Display component 410 may refer to, *e.g.,* a solid-state display that may have touch input capabilities. That is, display component 410 may include capabilities that may be shared with or replace those of input component 406.

Computer-readable medium 412 may refer to a separable machine-readable medium that is configured to store one or more programs that embody any of the functions or operations described above. That is, computer-readable medium 412, which may be received into or otherwise connected to a drive component of computing device 400, may store executable instructions to implement any of the functions or operations described above. These instructions may be complementary to or otherwise independent of those stored by memory 404.

Transceiver 414 may refer to a network communication link for computing device 400, configured as a wired network or direct-wired connection. Alternatively, transceiver 414 may be configured as a wireless connection, e.g., radio frequency (RF), infrared, Bluetooth, and other wireless protocols.

### Aspects:

Any of aspects 1 to 11 may be combined with any of aspects 12 to 20 and aspect 21, and any of aspects 12-20 may be combined with aspect 21.
Aspect 1. A computer-readable medium storing executable components that, upon execution, cause at least one processing device to perform a contaminant-risk analysis, the components comprising:
   an interface configured to receive parameters of a defined space from a user device;
   a CAD generator configured to generate a CAD model of the defined space based on the received parameters;
   a simulator configured to generate:
      a visual simulation of airflow within the CAD model of the defined space, wherein variations in velocity of the airflow are depicted in accordance with a first color-coding scale,
      a visual simulation of a flow of contaminants within the CAD model of the defined space, wherein variations in velocity and density of contaminants are depicted in accordance with a second color-coding scale, and
      a visual simulation of a flow of disinfectants within the CAD model of the defined space, wherein variations in velocity and density of disinfectants are depicted in accordance with a third color-coding scale; and
   a risk assessment generator configured to simultaneously project the visual simulation of the airflow, the visual simulation of the flow of contaminants, and the visual simulation of the flow of disinfectants onto the CAD model of the defined space.
Aspect 2. The computer-readable medium of Aspect 1, wherein the risk assessment generator is further configured to generate a contaminant risk assessment for the defined space based on a composite of the first color-coding scale, the second color-coding scale, and the third color-coding scale in the CAD model of the defined space.
Aspect 3. The computer-readable medium of Aspect 1 or Aspect 2, wherein the contaminant risk assessment is number-based.
Aspect 4. The computer-readable medium of any one of Aspects 1 to 3, wherein the contaminant risk assessment is color-based.
Aspect 5. The computer-readable medium of any one of Aspects 1 to 4, wherein the simulator is further configured to generate a revised CAD model of the defined space based on the generated contaminant risk assessment.
Aspect 6. The computer-readable medium of any one of Aspects 1 to 5, wherein the interface is configured to receive parameters of the defined space from a version of a scanning application running on the user device.
Aspect 7. The computer-readable medium of any one of Aspects 1 to 6, wherein the received parameters include dimensions of the defined space and placement of one or more air inlets and one or more air outlets within the defined space.
Aspect 8. The computer-readable medium of any one of Aspects 1 to 7, wherein the parameters received from the user via the interface further include a position of one or more people within the defined space.
Aspect 9. The computer-readable medium of any one of Aspects 1 to 8, wherein the parameters received from the user via the interface further include a position of one or more contaminant mitigation devices within the defined space.
Aspect 10. The computer-readable medium of any one of Aspects 1 to 9, wherein the revised CAD model includes re-positioning of one or more of a person, object, furniture, or a contaminant mitigation device within the CAD model of the defined space.
Aspect 11. The computer-readable medium of any one of Aspects 1 to 10, wherein the revised CAD model includes adding a contaminant mitigation device to the CAD model of the defined space.
Aspect 12. A risk-assessment system, comprising:
   a user device having a version of an application executing thereon;
   a service provider, having at least one processor communicatively connected to the application executing on the user device, the processor configured to:
      generate a CAD model of the defined space using the received parameters,
      generate a visual simulation of airflow within the CAD model of the defined space, wherein variations in velocity of the airflow are depicted in accordance with a first color-coding scale,
      generate a visual simulation of a flow of contaminants within the CAD model of the defined space, wherein variations in velocity and density of contaminants are depicted in accordance with a second color-coding scale,
      generate a visual simulation of a flow of disinfectants within the CAD model of the defined space, wherein variations in velocity and density of disinfectants are depicted in accordance with a third color-coding scale, and
      project the visual simulation of the airflow, the visual simulation of the flow of contaminants, and the visual simulation of the flow of disinfectants onto the CAD model of the defined space.
Aspect 13. The risk-assessment system of Aspect 12, wherein the service provider is further configured to generate a contaminant risk assessment for the defined space based on a composite of the first color-coding scale, the second color-coding scale, and the third color-coding scale in the CAD model of the defined space.
Aspect 14. The risk-assessment system of Aspect 12 or Aspect 13, wherein the contaminant risk assessment is either number-based or color-based.
Aspect 15. The risk-assessment system of any one of Aspects 12 to 14, wherein the service provider is further configured to generate a revised CAD model of the defined space based on the generated contaminant risk assessment.
Aspect 16. The risk-assessment system of any one of Aspects 12 to 15, wherein the received parameters include dimensions of the defined space and placement of one or more air inlets and one or more air outlets within the defined space.
Aspect 17. The risk-assessment system of any one of Aspects 12 to 16, wherein the parameters received from the user device further include a position of one or more people within the defined space.
Aspect 18. The risk-assessment system of any one of Aspects 12 to 17, wherein the parameters received from the user device further include a position of one or more contaminant mitigation devices within the defined space.
Aspect 19. The risk-assessment system of any one of Aspects 12 to 18, wherein the revised CAD model includes re-positioning of one or more of a person, object, furniture, or a contaminant mitigation device within the CAD model of the defined space.
Aspect 20. The risk-assessment system of any one of Aspects 12 to 19, wherein the revised CAD model includes adding at least one contaminant mitigation device to the CAD model of the defined space.
Aspect 21. A computer-readable medium storing executable instructions that, when executed, cause at least one processing device to perform a contaminant-risk analysis, the instructions comprising:
   receiving parameters of a defined space input by a user device, wherein the parameters include at least one or more of dimensions of the defined space, placement of at least one of an air inlet or an air outlet in the defined space, or placement of at least one contaminant mitigation devices in the defined space;
   generating a CAD model of the defined space based on the parameters;
   generating a visual simulation of airflow within the CAD model of the defined space, wherein variations in velocity of the airflow are depicted in accordance with a first color-coding scale;
   generating a visual simulation of a flow of contaminants within the CAD model of the defined space, wherein variations in velocity and density of contaminants are depicted in accordance with a second color-coding scale;
   generating a visual simulation of a flow of disinfectants within the CAD model of the defined space, wherein variations in velocity and density of disinfectants are depicted in accordance with a third color-coding scale; and
   producing a graphical risk assessment of the defined space by simultaneously projecting the visual simulation of the airflow, the visual simulation of the flow of contaminants, and the visual simulation of the flow of disinfectants onto the CAD model of the defined space.
Aspect 20. The computer-readable medium of Aspect 19, wherein the graphical risk assessment comprises a revised CAD model of the defined space that includes re-positioning of one or more of a person, object, furniture, or a contaminant mitigation device within the CAD model of the defined space.

From the foregoing, it will be appreciated that various embodiments of the present disclosure have been described herein for purposes of illustration, and that various modifications may be made without departing from the scope and spirit of the present disclosure. Accordingly, the various embodiments disclosed herein are not intended to be limiting, with the true scope and spirit being indicated by the following claims.

## Claims

1. A computer-readable medium storing executable components that, upon execution, cause at least one processing device to perform a contaminant-risk analysis, the components comprising:
an interface configured to receive parameters of a defined space from a user device;
a CAD generator configured to generate a CAD model of the defined space based on the received parameters;
a simulator configured to generate:
a visual simulation of airflow within the CAD model of the defined space, wherein variations in velocity of the airflow are depicted in accordance with a first color-coding scale,
a visual simulation of a flow of contaminants within the CAD model of the defined space, wherein variations in velocity and density of contaminants are depicted in accordance with a second color-coding scale, and
a visual simulation of a flow of disinfectants within the CAD model of the defined space, wherein variations in velocity and density of disinfectants are depicted in accordance with a third color-coding scale; and
a risk assessment generator configured to generate a contaminant risk assessment for the defined space based on generated output from the simulator.

2. The computer-readable medium of Claim 1, wherein the risk assessment generator is configured to generate the contaminant risk assessment for the defined space based on a composite of the first color-coding scale, the second color-coding scale, and the third color-coding scale in the CAD model of the defined space.

3. The computer-readable medium of either of Claims 1 or 2, wherein the contaminant risk assessment is number-based.

4. The computer-readable medium of any of Claims 1 to 3, wherein the contaminant risk assessment is color-based.

5. The computer-readable medium of any of Claims 1 to 4, wherein the simulator is further configured to generate a revised CAD model of the defined space based on the generated contaminant risk assessment.

6. The computer-readable medium of any of Claims 1 to 5, wherein the interface is configured to receive parameters of the defined space from a version of a scanning application running on the user device.

7. The computer-readable medium of any of Claims 1 to 6, wherein the received parameters include dimensions of the defined space and placement of one or more air inlets and one or more air outlets within the defined space.

8. The computer-readable medium of any of Claims 1 to 7, wherein the parameters received from the user via the interface further include a position of one or more people within the defined space.

9. The computer-readable medium of any of Claims 1 to 8, wherein the parameters received from the user via the interface further include a position of one or more contaminant mitigation devices within the defined space.

10. The computer-readable medium of any of Claims 5 to 9, wherein the revised CAD model includes re-positioning of one or more of a person, furniture, or a contaminant mitigation device within the CAD model of the defined space.

11. A risk-assessment system, comprising:
a user device having a version of an application executing thereon;
a service provider, having at least one processor communicatively connected to the application executing on the user device, the processor configured to:
generate a CAD model of the defined space using the received parameters,
generate a visual simulation of airflow within the CAD model of the defined space, wherein variations in velocity of the airflow are depicted in accordance with a first color-coding scale,
generate a visual simulation of a flow contaminants within the CAD model of the defined space, wherein variations in velocity and density of contaminants are depicted in accordance with a second color-coding scale,
generate a visual simulation of a flow of disinfectants within the CAD model of the defined space, wherein variations in velocity and density of disinfectants are depicted in accordance with a third color-coding scale, and
project the visual simulation of the airflow, the visual simulation of the flow of contaminants, and the visual simulation of the flow of disinfectants onto the CAD model of the defined space.

12. The risk-assessment system of Claim 11, wherein the service provider is further configured to generate a contaminant risk assessment for the defined space based on a composite of the first color-coding scale, the second color-coding scale, and the third color-coding scale in the CAD model of the defined space.

13. The risk-assessment system of either of Claims 11 or 12, wherein the contaminant risk assessment is either number-based or color-based.

14. The risk-assessment system of any of Claims 10 to 13, wherein the service provider is further configured to generate a revised CAD model of the defined space based on the generated contaminant risk assessment.

15. A computer-readable medium storing executable instructions that, when executed, cause at least one processing device to perform a contaminant-risk analysis, the instructions comprising:
receiving parameters of a defined space input by a user device, wherein the parameters include at least one or more of dimensions of the defined space, placement of at least one of an air inlet or an air outlet in the defined space, or placement of at least one contaminant mitigation devices in the defined space;
generating a CAD model of the defined space based on the parameters;
generating a visual simulation of airflow within the CAD model of the defined space, wherein variations in velocity of the airflow are depicted in accordance with a first color-coding scale;
generating a visual simulation of a flow of contaminants within the CAD model of the defined space, wherein variations in velocity and density of contaminants are depicted in accordance with a second color-coding scale;
generating a visual simulation of a flow of disinfectants within the CAD model of the defined space, wherein variations in velocity and density of disinfectants are depicted in accordance with a third color-coding scale; and
generating a contaminant risk assessment of the defined space based on the generated visual simulations.
